# FASCICULE DE BREVET EUROPEEN

(11) **EP 4 003 191 B1**
(45) Date de publication et mention de la délivrance du brevet: **23.10.2024**
(21) Numéro de dépôt: 20744083.5
(22) Date de dépôt: 29.07.2020
(51) Int. Cl.: A61B 17/12, A61F 2/90, A61B 90/00, A61B 34/10, A61B 17/00

(54) **DISPOSITIF INTRA-ANÉVRISMAL**
INTRA-ANEURYSMATISCHE VORRICHTUNG
INTRA-ANEURYSM DEVICE

(30) Priorité: 29.07.2019 EP 19305986
(43) Date de publication de la demande: 01.06.2022
(73) Titulaire: UNIVERSITE DE MONTPELLIER, 34090 Montpellier (FR); Centre Hospitalier Universitaire de Montpellier, 34090 Montpellier (FR); Centre national de la recherche scientifique, 75016 Paris (FR)
(72) Inventeur: BICHET, Lionel, 34070 Montpellier (FR); LOUCHE, Hervé, 34980 Saint-Gély-du-Fesc (FR); JOURDAN, Franck, 34000 Montpellier (FR); COSTALAT, Vincent, 34980 Saint-Gély-du-Fesc (FR)
(74) Mandataire: Cassiopi
(86) Numéro de dépôt international: PCT/EP2020/071434
(87) Numéro de publication internationale: WO 2021/018981

(56) Documents cités:
- WO-A1-2015/166013
- WO-A1-2017/156275
- WO-A1-2018/130624
- WO-A1-96/01599
- WO-A2-2006/052322
- WO-A2-2017/214431
- US-A1- 2002 198 561
- US-A1- 2006 206 200
- US-A1- 2012 197 283
- US-A1- 2017 079 662
- US-A1- 2019 008 522
- US-A1- 2019 105 054
- US-B1- 6 447 531

## Description

### Domaine technique de l'invention

L'invention a trait au domaine des dispositifs médicaux intra-anévrismaux. De façon plus précise, l'invention concerne les dispositifs intra-anévrismaux à diversion de flux.

### Arrière-plan technique

On appelle « anévrisme » une dilatation de la paroi d'une artère. La dilatation provoque une poche anévrismale qui communique avec l'artère grâce à une zone rétrécie connue des praticiens sous le nom « collet ».

La rupture d'un anévrisme, ou plus précisément de la poche anévrismale, peut avoir de lourdes conséquences parmi lesquelles, le décès. Plusieurs facteurs sont à l'origine des anévrismes. Il est à noter que les personnes âgées présentent une prévalence plus importante. Chez ces personnes, le risque de rupture est également plus important notamment en raison de pathologies connexes comme l'hypertension artérielle qui se manifeste sous la forme d'une augmentation de la pression artérielle.

Il s'agit par conséquent d'un problème de santé publique qu'il est nécessaire de traiter. Il existe différentes techniques de traitement d'un anévrisme.

Parmi les techniques de traitement d'un anévrisme, la technique dite « endovasculaire » se distingue des autres techniques par le fait qu'elle comprend une étape de traitement de l'anévrisme à l'aide d'un dispositif endovasculaire. Dans ce cas, l'anévrisme est abordé en suivant le trajet des artères. Un cathéter porteur (d'un dispositif intra-anévrismal) est introduit à travers les artères et dirigé sous contrôle radioscopique. Ceci peut aussi être un dispositif extra-anévrismal, intra-anévrismal ou les deux. Le dispositif est ensuite déposé dans l'anévrisme. En particulier, le dispositif est déposé dans l'artère porteuse de l'anévrisme dans le cas d'un dispositif extra-anévrismal ou dans l'anévrisme dans le cas d'un dispositif intra-anévrismal. Ceci permet d'isoler l'anévrisme du flux sanguin de l'artère afin d'éviter que la poche anévrismale rompt sous l'effet de la pression sanguine.

La mise en oeuvre de cette technique peut s'avérer particulièrement complexe selon la technique utilisée pour la conception du dispositif mais aussi la structure et la forme du dispositif, ceci à plus forte raison lorsque ce dispositif est de type intra-anévrismal.

Deux techniques de conception de dispositifs endovasculaires cérébraux existent.

Une première technique est dite « laser cut ». L'expression « laser cut » selon la terminologie anglo-saxonne désigne un procédé de découpe laser. Elle consiste à découper au laser un tube d'un alliage à mémoire de forme pour créer un réseau de cellules ouvertes ou fermées de forme généralement parallélépipédique. Cependant, les dispositifs fabriqués à partir de cette technique souffrent d'un affaissement dû aux courbures de l'artère, ce qui, outre le fait que cela représente un danger pour le patient, requiert, à terme, une nouvelle intervention sur le patient afin de pouvoir les remplacer. Or, ces interventions étant très lourdes pour le patient, il convient d'en limiter le nombre.

Une deuxième technique dite « tressée » consiste à créer une structure tressée à partir d'un ou plusieurs fils d'un alliage à mémoire de forme de sorte à former un réseau de cellules fermées. Cette technique, contrairement à la première technique décrite ci-dessus permet de concevoir des dispositifs repositionnables, flexibles et dont la structure est plus stable, ce qui permet leur installation pérenne au niveau l'artère à traiter. Les dispositifs fabriqués à partir de la technique tressée sont classiquement de forme tubulaire. Ils permettent de traiter de nombreuses autres pathologies.

Pour autant, l'usage de la technique tressée pour la conception de dispositifs intra-anévrismaux reste très limité, et pour cause ; bien que l'utilisation de la technique tressée soit bien adaptée à la conception de dispositifs de formes simples à réaliser, par exemple de forme tubulaire, elle se prête beaucoup moins à celle de dispositifs de formes plus complexes qui requièrent des outils de fabrication spécifiques et dont le temps de fabrication peut s'avérer particulièrement long. La structure en forme d'anse résultant de la configuration de l'anévrisme par rapport à l'artère, de même que la présence de bifurcations à proximité immédiate de l'anévrisme, rendent en effet l'utilisation de la technique tressée pour la conception des dispositifs intra-anévrismaux moins courante, sans pour autant en exclure l'usage.

Parmi les techniques tressées, on trouve les dispositifs de type contour. Ces dispositifs comprennent une tête destinée à être insérée dans l'anévrisme et formant une structure tressée faite d'une pluralité de fils. La tête comprend une extrémité nodale au niveau de laquelle les fils sont reliés par sertissage et une extrémité distale au niveau de laquelle les fils font des « allers-retours », telles que des boucles. Les fils s'étendent de manière ininterrompue au niveau des boucles et ne sont reliés entre eux qu'au niveau de l'extrémité nodale. En plus de la bague nécessaire au sertissage des fils au niveau de cette extrémité nodale, un très grand nombre de fils est nécessaire pour assurer la stabilité structurelle des dispositifs contours. Ces dispositifs sont donc très encombrants et l'accès à certains anévrismes situés loin dans les artères est compliqué, voire impossible.

Parmi les techniques tressées, on trouve des « coils ». L'expression « coils » est un terme anglais désignant une petite spire de métal utilisée pour permettre l'obturation d'un vaisseau. En effet de nombreux dispositifs intra-anévrismaux utilisent des coils pour remplir la poche anévrismale de l'anévrisme à traiter afin de limiter l'afflux de sang vers la coupole et donc l'anévrisme. L'opération visant à remplir la poche anévrismale des coils est appelée « coiling ». Cette opération peut s'avérer relativement longue puisqu'il faut remplir l'intégralité de la poche anévrismale pour que le dispositif soit pleinement fonctionnel.

Par exemple, le document WO2018/130624A1 décrit un dispositif anévrismal à diversion de flux. En outre, le document US2002/198561 A1 décrit une méthode pour former des dispositifs intravasculaires à partir d'un tissu métallique résilient. De plus, le document WO 2006/052322 décrit un autre exemple de dispositif intra-anévrismal à structure tressée de type coils. Le dispositif intra-anévrismal divulgué comprend un stent permettant l'ancrage du dispositif dans une artère source, porteuse de l'anévrisme. Le stent est un terme anglais couramment utilisé dans le domaine médical pour désigner un tube de section sensiblement circulaire. L'ancrage se fait par endothélialisation du stent sur la paroi interne de l'artère. Le dispositif comprend une tête, généralement en forme de coupole destinée à loger à l'intérieur de la poche anévrismale. Entre la coupole et le stent, le dispositif comprend en outre une portion intermédiaire amincie qui délimite la coupole et le stent.

Cependant, le temps de déploiement de ce dispositif est très long et la structure n'est pas suffisamment stable pour permettre son déploiement dans un temps plus réduit.

### Résumé de l'invention

L'invention est définie dans la revendication indépendante 1. Certaines caractéristiques optionnelles de l'invention sont définies dans les revendications dépendantes. Un objectif de l'invention est de résoudre l'un au moins des problèmes précités

À cet effet, il est proposé un dispositif intra-anévrismal pour le traitement d'un anévrisme, comprenant une structure tressée, à mailles, réalisée avec une pluralité de fils, la structure tressée comprenant une tête destinée à être insérée dans l'anévrisme, la tête étant apte à réduire significativement un flux sanguin dans l'anévrisme, ladite tête présentant une forme de coupole.

Le dispositif est caractérisé en ce que la structure tressée comprend au niveau d'une extrémité proximale de ladite structure tressée un noeud relié à la tête et en ce que, au niveau de son extrémité distale opposée à ladite extrémité proximale, la tête est formée d'extrémités de fils dont certaines au moins sont liées mécaniquement entre elles, les extrémités des fils liés mécaniquement étant parallèles et liés deux à deux.

Le dispositif selon l'invention est ainsi configuré de sorte à pouvoir être transporté jusqu'à la zone affectée au moyen d'un micro-cathéter dont les dimensions sont beaucoup plus faibles que celles du dispositif sans que cela ne s'avère dommageable pour la tenue structurelle de la tête dont les dimensions sont plus importantes que celles du micro-cathéter puisqu'elle est destinée à être insérée dans l'anévrisme.

En effet, le fait que la structure tressée comprenne au niveau d'une extrémité proximale de ladite structure tressée un noeud relié à la tête et que, au niveau de l'extrémité distale de ladite structure tressée, la tête soit formée d'extrémités de fils dont certaines au moins sont liées mécaniquement entre elles permet de préserver la tenue structurelle de la tête et d'améliorer la stabilité structurelle du dispositif lors de la phase de déploiement. En effet, on évite ainsi la déformation structurelle du dispositif et l'éloignement des fils les uns par rapport aux autres qui se produirait lors du passage dans le micro-cathéter. Une fois que le dispositif est correctement positionné au sein de l'anévrisme, la phase de déploiement est achevée et le dispositif est immédiatement fonctionnel. Aucun temps supplémentaire n'est nécessaire pour un quelconque remplissage de la poche anévrismale comme c'est le cas avec les dispositifs de type coils.

En outre, le dispositif selon l'invention présente un encombrement très réduit ce qui lui permet d'être implanté plus loin dans les artères touchées et lui permet de traiter un plus grand nombre d'anévrismes. Cela tient d'une part au fait que le noeud relié à la tête est formé par la structure tressée elle-même, ce qui évite d'avoir nécessairement recours à une bague, et d'autre part au fait que certaines des extrémités des fils étant liées mécaniquement entre elles au niveau de l'extrémité de la tête opposée au noeud, la stabilité structurelle du dispositif tient à ces liaisons et non au nombre de fils, qui peut alors être très réduit.

Selon différentes caractéristiques de l'invention qui pourront être prises ensemble ou séparément :
- les mailles de la structure tressée sont plus denses au niveau de l'extrémité proximale de ladite tête qu'au niveau de l'extrémité distale de ladite tête ;
- la tête présente une géométrie sensiblement demi-sphérique tournée vers l'extérieur, l'extrémité distale de ladite tête présentant une section sensiblement circulaire de diamètre D2 ;
- le noeud présente une section sensiblement circulaire dont un diamètre D3 est inférieur au diamètre de la tête D2 ;
- le nombre de fils est compris entre 4 et 250 de préférence compris entre 16 et 32 ;
- les fils présentent un diamètre compris entre 10 µm et 500 µm ;
- les fils se superposent au niveau de zones d'intersection ;
- lesdits fils sont liés mécaniquement au niveau desdites zones d'intersection ;
- certaines au moins des extrémités de fils sont liées mécaniquement entre elles par l'intermédiaire d'une portion de liaison faite d'une partie d'un premier fil et d'une partie d'un deuxième fil ;
- les fils sont en matériau biocompatible de préférence du nitinol, platine ou titane ;
- au moins un fil est radio-opaque ;
- le dispositif comprend une portion proximale et destinée à positionner le dispositif ;
- ledit noeud relie la portion proximale à la tête ;
- les mailles de la structure tressée au niveau du noeud sont plus denses que les mailles de la structure tressée au niveau de l'extrémité proximale de la tête ;
- ledit noeud est formé par une bague de sertissage reliant des extrémités de fils entre elles ;
- ledit noeud est formé par des extrémités de fils 6c soudées entre elles.

### Brève description des figures

D'autres objets et caractéristiques de l'invention apparaîtront plus clairement dans la description qui suit, faite en référence aux figures annexées, dans lesquelles :
[Fig. 1] la figure 1 illustre une vue en perspective d'un dispositif selon un premier mode de réalisation de l'invention ;
[Fig. 2] la figure 2 illustre une vue en perspective d'un dispositif selon un second mode de réalisation de l'invention ;
[Fig. 3a] La figure 3a illustre une vue latérale du dispositif durant la phase d'initiation de son déploiement au sein de l'anévrisme ;
[Fig. 3b] La figure 3b illustre une vue en perspective du dispositif durant la phase d'apposition sur les parois de l'anévrisme ;
[Fig. 3c] La figure 3c illustre une vue en perspective du dispositif durant la phase finale de son déploiement au sein de l'artère ;
[Fig. 4] La figure 4 illustre une vue de face du dispositif selon l'invention après déploiement au sein d'une artère reconstituée ;
[Fig. 5] La figure 5 illustre le résultat d'une simulation numérique de déploiement du dispositif selon l'invention en vue de face ;
[Fig. 6a] La figure 6a illustre une vue rapprochée du dispositif selon l'invention lors du procédé de liaison des extrémités des fils selon une première variante ;
[Fig. 6b] La figure 6b illustre une vue rapprochée du dispositif selon l'invention lors du procédé de liaison des extrémités des fils selon une deuxième variante ;
[Fig. 6c] La figure 6c illustre une portion de liaison du dispositif de la figure 6a ;
[Fig. 6d] La figure 6d illustre une portion de liaison du dispositif de la figure 6b ;
[Fig. 7] La figure 7 illustre un procédé de liaison des extrémités des fils ;

### Description détaillée de l'invention

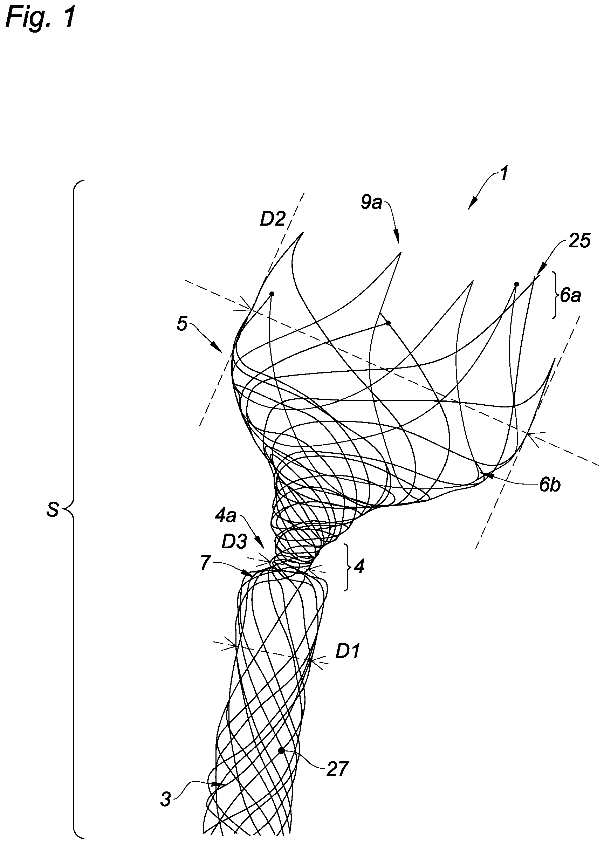

En référence à la figure 1, l'invention concerne un dispositif 1 intra-anévrismal pour le traitement endovasculaire d'un anévrisme 2. En d'autres termes, le dispositif 1 selon l'invention est apte et destiné à être implanté au sein dudit anévrisme 2. Le dispositif 1 tel qu'illustré à la figure 1 est au repos.

On rappelle que l'anévrisme résulte de la dilation de la paroi d'un vaisseau, sanguin, porteur. Ce vaisseau est, typiquement, une artère. La dilatation provoque une poche anévrismale 8 qui communique avec l'artère grâce à une zone rétrécie connue des praticiens sous le nom « collet ».

L'anévrisme 2 dont il s'agit peut présenter toute morphologie. Il peut s'agir d'un anévrisme à collet large ou non. Il en est de même pour le vaisseau porteur qui peut présenter des dimensions très variées selon la partie du corps concernée, d'autres facteurs étant à prendre en compte.

En outre, l'anévrisme 2 dont il s'agit peut être localisé dans n'importe quelle partie du corps comprenant un vaisseau touché. Par exemple, il peut être localisé dans l'aorte, ce qui en fait un anévrisme aortique. Il peut également être localisé dans une artère du cerveau, ce qui en fait un anévrisme cérébral ou intra-crânien. Les anévrismes cérébraux sont souvent difficilement accessibles, ce qui rend le déploiement d'un dispositif endovasculaire et donc leur traitement par voie endovasculaire particulièrement complexe voire même impossible à réaliser dans certains cas. Comme cela sera vu dans les sections qui suivent, le dispositif 1 intra-anévrismal selon l'invention est particulièrement bien adapté au traitement des anévrismes cérébraux du fait de son déploiement facilité et de son faible encombrement.

Le dispositif 1 intra-anévrismal est plus particulièrement un dispositif à diversion de flux. Les dispositifs de ce type se distinguent des dispositifs à coils et à cage intra-anévrismale essentiellement en ce que le traitement de l'anévrisme ne se fait pas par remplissage de la poche anévrismale avec des coils ou avec un matériel d'embolisation, selon le cas, mais par un traitement de l'artère porteuse de l'anévrisme.

Le dispositif 1 selon l'invention comporte une tête 5 formant une structure tressée S, à mailles, réalisée avec une pluralité de fils 6.

La tête 5 est destinée à être insérée dans l'anévrisme 2. Elle a pour fonction de réduire significativement le flux sanguin dans l'anévrisme 2. L'agencement des fils 6 au niveau de l'extrémité sera évoqué de manière plus détaillée dans la suite. Cela étant, on peut sans doute préciser à ce stade que les extrémités 6a des fils forment un pourtour de la tête 5.

Selon un aspect de l'invention, la structure tressée S comprend, au niveau d'une extrémité proximale de ladite structure tressée S, un noeud 4a relié à la tête 5. Cela permet d'accroître la tenue structurelle de la tête 5 lors de la phase de déploiement du dispositif 1 intra-anévrismal dans l'artère porteuse. Le noeud 4a permet de stabiliser la structure tressée S au niveau de la tête 5, c'est-à-dire entre l'extrémité proximale de la structure tressée S et l'extrémité distale de ladite structure tressée S, sans empêcher que ladite tête 5 ne soit mobile par rapport au noeud 4a.

Selon une variante de réalisation de l'invention, les mailles de la structure tressée S au niveau du noeud 4a sont plus denses que les mailles de la structure tressée au niveau de l'extrémité proximale de la tête. La densité est définie ici comme le nombre de maille(s) par unité de surface en unité arbitraire. Les mailles de la structure tressée S sont dites plus denses car, au niveau dudit noeud, elles sont plus nombreuses par unité de surface. Autrement dit, le maillage de la structure tressée S, au niveau dudit noeud 4a, est « fins et serré » par opposition à un maillage lâche « plus large et détendu » plus susceptible de se déformer ou de perdre sa structure si aucun moyen n'est prévu pour le préserver. Cette configuration est particulièrement bien adaptée à un dispositif 1 qui est muni en plus de la tête 5 et du noeud 4a, d'une portion proximale 3, par exemple un stent, comme nous le verrons dans la suite (Figure 1).

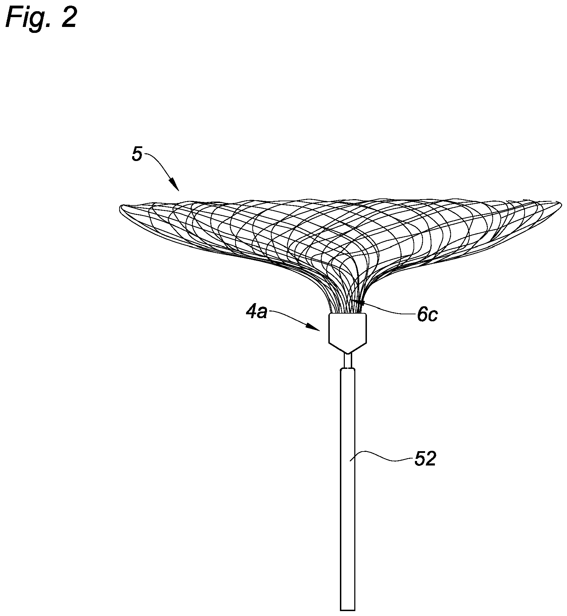

Selon une autre variante de réalisation de l'invention, la structure tressée S est formée, au niveau dudit noeud 4a, d'extrémités 6c de fils reliées, c'est-à-dire réunies. Cette configuration est particulièrement bien adaptée à un dispositif 1 intra-anévrismal comprenant uniquement une tête 5 et un noeud 4a (Figure 2). En effet, dans ce cas, le noeud 4a relie alors la pluralité de fils 6. Dans une première configuration, ledit noeud 4a peut être formé par une bague de sertissage reliant des extrémités de fils 6c entre elles. Alternativement, dans une seconde configuration, ledit noeud 4a peut être formé par des extrémités de fils 6c soudées entre elles.

Dans une variante comme dans une autre, le noeud 4a présente une section de diamètre D3 bien inférieur à celui de la tête 5.

En outre, le fait que le noeud 4a est relié à la tête 5, au niveau de l'extrémité proximale de ladite structure tressée S, permet d'accroître la maniabilité de la tête 5 et réduit ainsi le temps d'intervention. En effet, durant la phase de déploiement, le dispositif 1, qui se trouve initialement complètement inséré dans le micro-cathéter 50, est poussé par un poussoir 52 du micro-cathéter 50 en direction de la zone à traiter. La présence du noeud 4a permet ainsi d'éviter que le dispositif 1 ne perde sa tenue structurelle sous l'effet de la contrainte exercée par le poussoir 52 du micro-cathéter 50 sur ledit noeud 4a et donc sur la tête 5 qui se trouve dans son prolongement.

Selon un autre aspect de l'invention, au niveau de l'extrémité distale opposée à ladite extrémité proximale de ladite structure tressée S, la tête 5 est configurée de telle sorte que certaines au moins des extrémités 6a de fils sont parallèes et liées mécaniquement entre elles. Cela permet de préserver la tenue structurelle du dispositif 1 lors de la phase de déploiement du dispositif 1 intra-anévrismal dans l'artère porteuse. En effet, lorsque le dispositif 1 est inséré dans le micro-cathéter 50 aux fins de son déploiement et de son placement dans la zone à traiter, il subit d'importantes contraintes dues aux faibles dimensions du micro-cathéter 50 par rapport à celles du dispositif 1 intra-anévrismal. En effet, le diamètre intérieur du micro-cathéter 50 est typiquement compris entre 0,4 mm et 2 mm. Dans une telle situation, la tête 5 subit des contraintes très importantes puisqu'étant destinée à être insérée dans l'anévrisme 2 elle présente de grandes dimensions. La liaison de certaines extrémités 6a des fils situées au niveau de l'extrémité distale de la structure tressée S permet d'améliorer la stabilité structurelle de la tête 5 et de préserver la tenue structurelle du dispositif 1 en évitant la déformation de la tête 5 et l'éloignement des fils 6 les uns par rapport aux autres.

En outre, dans cette configuration, il n'est pas nécessaire de prévoir un temps supplémentaire pour le remplissage de la poche anévrismale avec des coils ou avec un matériel d'embolisation comme c'est typiquement le cas dans les dispositifs à coils et à cage intra-anévrismale respectivement puisque la structure initiale du dispositif est préservée après la phase de déploiement. Cela réduit la durée de l'intervention sur le patient.

De préférence, chacune desdites extrémités 6a des fils est liée à au moins une autre desdites extrémités 6a des fils. Autrement dit, les extrémités 6a des fils sont liées mécaniquement au moins deux à deux. La tête 5 présente l'apparence d'un nénuphar ayant des pétales de forme conique. Dans cette configuration, les extrémités étant liées mécaniquement au moins, deux à deux, de manière continue sur tout le pourtour de l'extrémité de la tête, la structure des mailles est préservée. Incidemment, cela confère davantage de tenue structurelle à la tête 5 et de stabilité structurelle au dispositif 1 intra-anévrismal lors de la phase de déploiement.

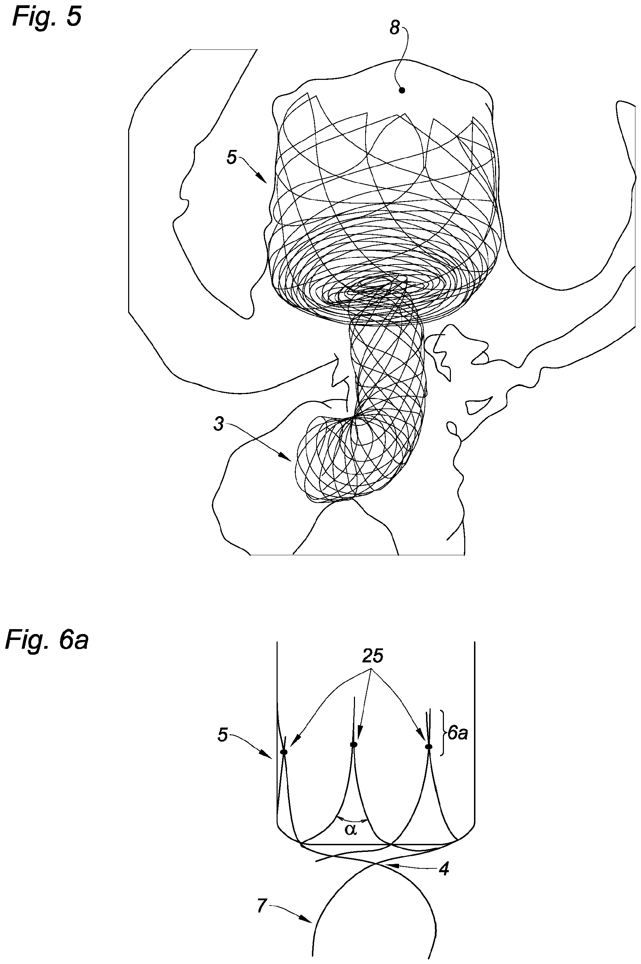

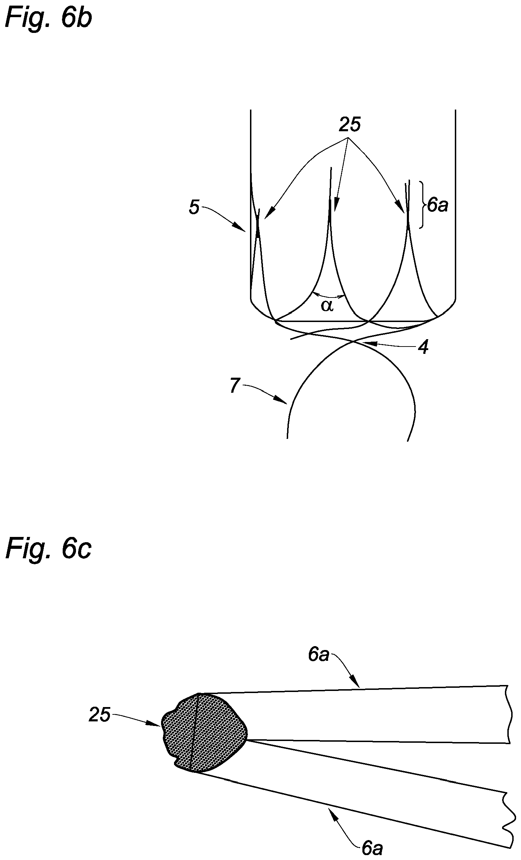

De préférence, les extrémités 6a des fils sont liées mécaniquement entre elles par l'intermédiaire d'une portion 25 de liaison. La portion 25 s'étend sensiblement au niveau de la portion périphérique des fils 6. Il ne s'agit pas nécessairement exactement du point le plus extrême desdits fils 6 mais d'une portion s'étendant depuis ce point jusqu'à une zone périphérique desdits fils 6. Les figures 6a et 6c illustrent une telle configuration.

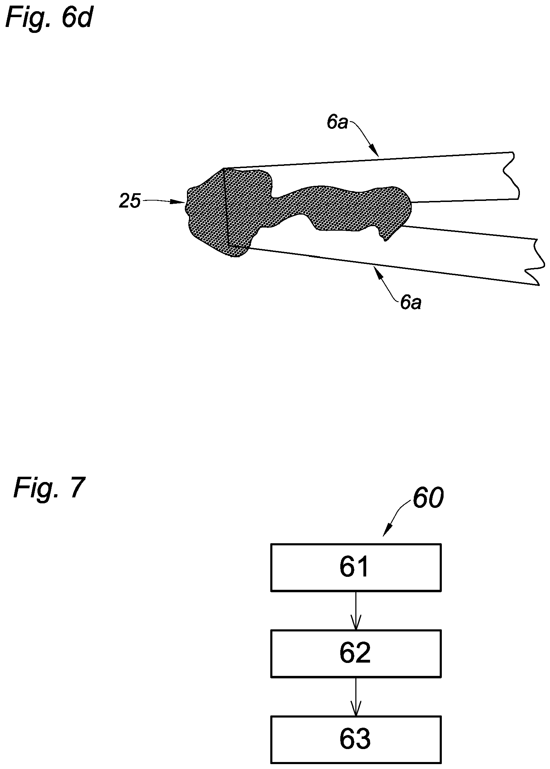

Cela étant dit, la portion 25 de liaison n'est pas nécessairement un point. La portion 25 de liaison peut également être linéaire et non ponctuelle. Autrement dit, dans un tel cas les extrémités 6a des fils ne sont pas liées mécaniquement entre elles en un unique point mais sur une ligne, ou encore un cordon. De préférence, la longueur de ladite portion 25 de liaison est comprise entre 0,05 mm et 1 mm. La liaison mécanique entre les extrémités 6a des fils est alors plus résistante, et comme cela sera vu plus loin même sans ajout de matière. Les figures 6b et 6d illustrent une telle configuration.

Ainsi, le noeud 4a étant relié à la tête 5 et la tête 5 étant configurée de telle sorte que certaines au moins des extrémités 6a de fils situées au niveau de l'extrémité distale de la structure tressée S sont liées mécaniquement entre elles d'autre part, la stabilité structurelle du dispositif 1 est préservée durant la phase de déploiement du dispositif 1 dans l'anévrisme 2 et le temps d'intervention sur le patient est réduit. Cela confère avantageusement au dispositif 1 selon l'invention un encombrement réduit en comparaison avec les dispositifs de type contour connu de l'art antérieur. En effet, la stabilité structurelle du dispositif tient aux liaisons existant au noeud 4a et à certaines au moins des extrémités 6a de fils 6 et non au nombre de fils. Ce dernier peut alors être très faible.

Le dispositif 1 intra-anévrismal selon l'invention peut présenter plusieurs configurations selon qu'il est au repos, en phase de déploiement ou encore en usage. L'aptitude du dispositif 1 à passer d'une configuration à une autre tient d'une part dans les propriétés de la matière utilisée pour sa conception et d'autre part dans sa structure à base de micro-fils tressés. Pour autant, comme nous le verrons en détails dans les sections qui suivent, cette aptitude du dispositif 1 selon l'invention à passer d'une configuration à une autre n'affecte pas sa bonne tenue structurelle, en particulier durant la phase de déploiement.

En référence à la figure 1, il est décrit dans ce qui suit la configuration du dispositif 1 intra-anévrismal selon un premier mode de réalisation de l'invention au repos, c'est à dire la configuration du dispositif 1 en l'absence de toute pression sur ledit dispositif 1.

Le dispositif 1 selon ce premier mode de réalisation de l'invention peut comprendre en plus de la tête 5, une portion proximale 3, qui est par exemple un stent. La portion proximale 3, le noeud 4a et la tête 5 forment alors la structure tressée S.

Il s'agit dans ce mode de réalisation particulier d'associer les avantages de la technique intra-anévrismale et celles des stents classiques.

Comme mentionné précédemment, la structure tressée S est réalisée avec une pluralité de fils 6. Les fils 6 sont agencés de sorte à former une pluralité de cellules de forme sensiblement parallélépipédique. Chaque cellule ou maille comprend une zone centrale dépourvue de matière, c'est-à-dire non couverte par les fils 6 et un pourtour délimité par les fils 6. Les cellules sont ainsi dites « fermées ». Selon la surface des cellules, on associe la structure tressée S à un maillage fin ou un maillage large. En l'occurrence, un maillage fin comporte des cellules occupant une plus faible surface en comparaison avec un maillage large. Précisons que, sur ce mode de réalisation particulier, la portion proximale 3, la tête 5 et le noeud 4a peuvent être formés avec les mêmes fils 6, ce qui contribue à améliorer la tenue structurelle du dispositif 1 intra-anévrismal, mais qu'ils ne présentent pas nécessairement le même type de maillage.

Les fils 6 présentent des dimensions longitudinales millimétriques et des dimensions diamétrales micrométriques. Plus précisément, les fils 6 présentent un diamètre compris entre 10 et 500 µm. Ce diamètre est avantageusement inférieur ou égal à 50 µm pour les anévrismes cérébraux. Cela étant, il est d'environ 500 µm pour les anévrismes thoraciques. De préférence, le nombre de fils 6 est compris entre 4 et 250 selon les dimensions du dispositif, et de fait les dimensions de l'anévrisme et de l'artère porteuse. Encore plus préférentiellement, le nombre de fils varie entre 16 et 32. Un tel nombre de fils 6 permet avantageusement de faciliter l'insertion du dispositif 1 intra-anévrismal dans un micro-cathéter 50 servant au déploiement du dispositif 1 dans la zone artérielle touchée par l'anévrisme. Cela permet également d'obtenir une structure tressée S de flexibilité et de maniabilité suffisante pour une insertion dans les parties du corps les plus difficilement accessible. Toutefois, précisons que le nombre de fils 6 dépend substantiellement du diamètre desdits fils. Ainsi, si le diamètre des fils 6 est de 100 µm, le nombre de fils ne pourra excéder 4. En revanche, si le diamètre des fils 6 est de 25 µm, le nombre de fils peut alors être bien supérieur, par exemple 16.

À ce propos, le dispositif 1 intra-anévrismal est avantageusement réalisé dans un alliage de nickel et de titane communément appelé nitinol. Outre la maniabilité qu'offre cette matière, elle présente également une excellente mémoire de forme et une très bonne élasticité, ce qui lui permet de se conformer à la morphologie de la zone dans laquelle elle se trouve.

Avantageusement, au moins un fil est radio-opaque. Ceci permet de visualiser le fil sur un écran, pendant l'intervention permettant également d'observer l'état d'ouverture du dispositif 1.

La portion proximale 3 est apte et destinée à positionner le dispositif 1 intra-anévrismal dans l'artère porteuse. Dans ce mode de réalisation particulier, la portion proximale 3 présente au repos une section sensiblement circulaire de diamètre D1 approprié pour permettre l'ancrage/le positionnement du dispositif 1 intra-anévrismal dans l'artère touchée par l'anévrisme 2. La portion proximale 3 présente donc une forme sensiblement tubulaire.

Le noeud 4a est, quant à lui, avantageusement positionné au niveau d'une portion intermédiaire 4 du dispositif 1. La portion intermédiaire 4 s'étend à partir d'une extrémité 7 supérieure de la portion proximale 3 jusqu'à une base de la tête 5. Le noeud 4a présente une section circulaire de diamètre, D3, inférieur au diamètre D1 de la portion proximale 3, sans pour autant que cela n'empêche l'écoulement de sang au travers dudit noeud 4a. À titre d'exemple, le diamètre D3 du noeud 4a peut être deux fois inférieur au diamètre D1 de la portion proximale 3. Cela permet un bon transit du flux sanguin dans les artères sous-jacentes à l'anévrisme 2.

Comme mentionné précédemment, la portion proximale 3, le noeud 4a et la tête 5 sont avantageusement formés avec les mêmes fils 6. Toutefois, le maillage de la structure tressée S au niveau du noeud 4a est plus étroit. Cela permet, en plus des avantages vus précédemment, de s'affranchir de l'utilisation d'un moyen de serrage additionnel tel qu'une bague.

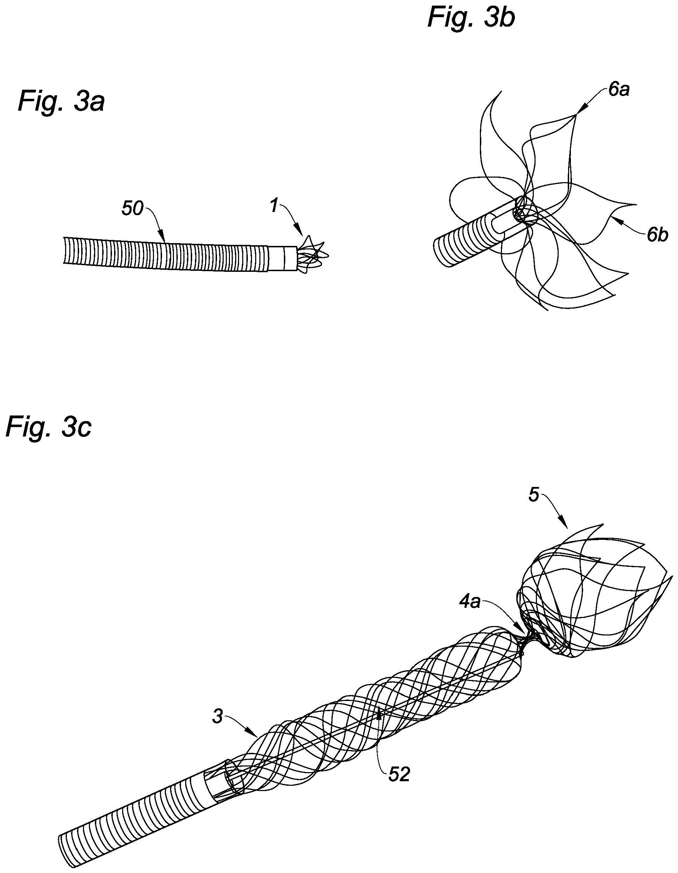

En référence au premier (figure 1) et deuxième (figure 2) mode de réalisation de l'invention, la tête 5 présente une forme de coupole concave dont la concavité 9a est tournée vers l'extérieur. Autrement dit, la tête 5 se présente sous forme d'une demi-sphère ou d'une sphère tronquée ou d'un évasement ou d'un ovoïde tronqué tourné(e) vers l'extérieur. L'extrémité distale de la tête 5 présente une section sensiblement circulaire dont un diamètre D2 augmente généralement en direction de son extrémité. En effet, au niveau de son extrémité distale, le diamètre de la tête 5 diminue légèrement du fait de la légère courbure vers l'intérieur des pétales. C'est au niveau d'une section située entre son extrémité distale et une partie médiane que le diamètre D2 de la tête 5 est maximum. Cela étant, il reste dans l'ensemble nettement supérieur en comparaison avec le diamètre au niveau du noeud, et même au diamètre D1 de la portion proximale 3 pour ce qui est du mode de réalisation illustré à la figure 1. Une fois le dispositif 1 installé sur un patient, la concavité 9a fait face à la poche 8 anévrismale ainsi qu'illustré sur la figure 3. Pour autant, cela n'empêche pas le pourtour de l'extrémité libre de la tête 5 d'être grossier et sinueux, c'est-à-dire qu'il n'est pas régulier ou droit.

Au niveau de l'extrémité proximale de ladite tête, les mailles de la structure tressée S sont plus denses qu'au niveau de l'extrémité distale de ladite tête, c'est-à-dire les mailles dont plus lâches. Ainsi, les mailles de la tête 5 présentent des dimensions croissantes à mesure qu'elles sont éloignées de la portion intermédiaire 4, notamment du noeud 4a. Autrement dit, au niveau de son extrémité, le maillage de la tête 5 est donc beaucoup plus large que le maillage de la tête 5 à proximité immédiate de la portion intermédiaire 4. Il est aussi plus large que celui de la portion proximale 3, de la portion intermédiaire 4 et donc du noeud 4a. De manière particulièrement avantageuse, un tel maillage permet en outre de favoriser un plaquage de la tête 5 contre les parois de l'anévrisme 2 lorsque le dispositif 1 intra-anévrismal est en usage, i.e. après déploiement. L'usage de fils 6 en nitinol contribue certes à un tel plaquage, mais dans une bien moindre mesure, le nitinol ayant une force radiale relativement faible.

On peut également préciser que le maillage de la structure tressée S au niveau de la tête 5 étant détendu, le risque de rétractation du maillage est considérablement réduit. Incidemment, cela permet de réduire le risque de rupture retardé de l'anévrisme 2. En effet, une éventuelle rétractation du maillage de la tête 5 aurait pour conséquence de laisser un espace libre entre la tête 5 et l'anévrisme 2 dans lequel le sang pourrait circuler en exerçant sans aucune contrainte une pression sur les parois de l'anévrisme 2, ce qui à terme annulerait les effets. En outre, le maillage lâche au niveau de la tête 5 permet d'accroître substantiellement la flexibilité de la tête 5, ce qui permet avantageusement de traiter des anévrismes de géométries et de dimensions plus variées et donc de traiter un plus grand nombre d'anévrismes.

Ceci étant dit, l'intérêt de la solution de l'invention apparaît encore mieux compte-tenu du maillage moins dense de la structure tressée S au niveau de la tête 5. En effet, le fait que le noeud 4a soit relié à la tête 5 stabilise le maillage au niveau de l'extrémité proximale de la structure tressée, c'est-à-dire du côté noeud 4a de la tête 5. Concomitamment, à l'extrémité distale de la structure tressée, c'est-à-dire du côté de la tête 5 opposé au noeud 4a, la présence de liaison entre certaines extrémités 6a des fils permet d'éviter que le maillage initial ne s'altère complètement par le désassemblage des mailles qui le composent.

En référence aux figures 2a à 2c, les principales phases de déploiement du dispositif 1 intra-anévrismal sont illustrées.

La figure 3a illustre le dispositif 1 intra-anévrismal lors de la phase initiale du déploiement. Dans cette configuration, on distingue à peine la tête 5, le dispositif 1 étant quasi intégralement inséré dans le micro-cathéter 50. Les extrémités 6a des fils sont visibles.

La figure 3b illustre le dispositif 1 intra-anévrismal lors d'une phase plus avancée dans le déploiement. Dans cette configuration, la portion intermédiaire 4, le noeud 4a et la portion proximale ne sont toujours pas visibles. Le micro-cathéter 50 continue d'exercer une pression sur la portion de la tête 5 située à proximité de la portion intermédiaire. Néanmoins, on distingue mieux la structure en forme de pétales résultant de la liaison d'au moins certaines extrémités 6a des fils entre elles.

Des portions 6b disjointes des fils 6 apparaissent moins rapprochées les unes des autres en comparaison de la configuration qu'elles adoptent lorsque le dispositif 1 est au repos (Figure 1). Même en étant soumises à la contrainte générée par l'écartement des portions 6b disjointes, les liaisons 25 sont conservées. Ceci s'explique par le procédé de liaison des extrémités 6a des fils qui sera décrit de manière plus détaillée dans la suite.

À ce propos, si certaines des extrémités 6a des fils sont liées mécaniquement, elles ne doivent l'être qu'au moins pendant la phase de déploiement. En effet, la perte de tenue structurelle n'est dommageable que si elle se produit avant que la tête 5 ait pu être correctement positionnée au sein de l'anévrisme 2. Or, c'est justement pendant la phase de déploiement que le risque de perte de la tenue structurelle est le plus élevé du fait de la différence de dimension qui existe entre le dispositif 1 intra-anévrismal et le micro-cathéter 50, qui pour rappel présente des dimensions comprises entre 0,4 et 2 mm. Cela étant rappelé, si les extrémités 6a des fils viennent à se désolidariser les unes des autres après le déploiement du dispositif 1, cela n'a pas d'incidence sur le fonctionnement du dispositif 1.

La figure 3c illustre le dispositif 1 intra-anévrismal lors de la phase finale du déploiement. Dans cette configuration, la quasi-totalité du dispositif 1 est déployée. On distingue nettement la portion proximale 3, le noeud 4a et la tête 5. Plus aucune pression ne s'exerce sur la tête 5 de sorte qu'elle retrouve sa configuration de repos. La tenue structurelle de la tête 5 a été préservée puisque le maillage de la structure tressée S est intact au niveau de la tête 5. Cela n'aurait pas été le cas si le noeud 4a n'était pas relié à la tête 5. En effet, dans ce cas, sous l'effet de la poussée du poussoir 52 du micro-cathéter, le noeud 4a puis la tête 5 auraient subi des déformations. Cela n'aurait pas non plus été le cas si certaines des extrémités 6a des fils n'avaient pas été liées mécaniquement entre elles. En effet, dans ce cas, sous l'effet de la poussée du le poussoir 52 du micro-cathéter et de la pression exercée par les parois internes du micro-cathéter 50 (comme illustré à la fig. 2b), le maillage de la structure tressée S se serait désagrégé.

En outre, la portion proximale 3 dont le diamètre D1 est supérieur au diamètre du micro-cathéter 50 et sur le pourtour duquel des pressions s'exerçaient a également retrouvé sa configuration de repos.

Précisons que d'autres liaisons peuvent être prévues afin d'améliorer encore plus la stabilité structurelle du dispositif 1. La structure tressée S peut comprendre, à certaines intersections entre les fils 6, des points 27 de liaison au niveau desquels les fils 6 sont liés mécaniquement. Lesdits points 27 de liaison sont situés à un ou plusieurs emplacements appropriés dans la structure tressée S, c'est-à-dire au choix dans le maillage de la portion proximale 3, le maillage de la portion intermédiaire 4 ou encore dans le maillage de la tête 5. L'intérêt est moindre pour ce qui concerne le maillage de la portion intermédiaire 4 qui est avantageusement relativement fin. Cela dit, au niveau de la portion proximale 3 et de la tête 5, cela peut être très avantageux afin de stabiliser encore plus la structure tressée S lors du passage du dispositif dans le micro-cathéter 50.

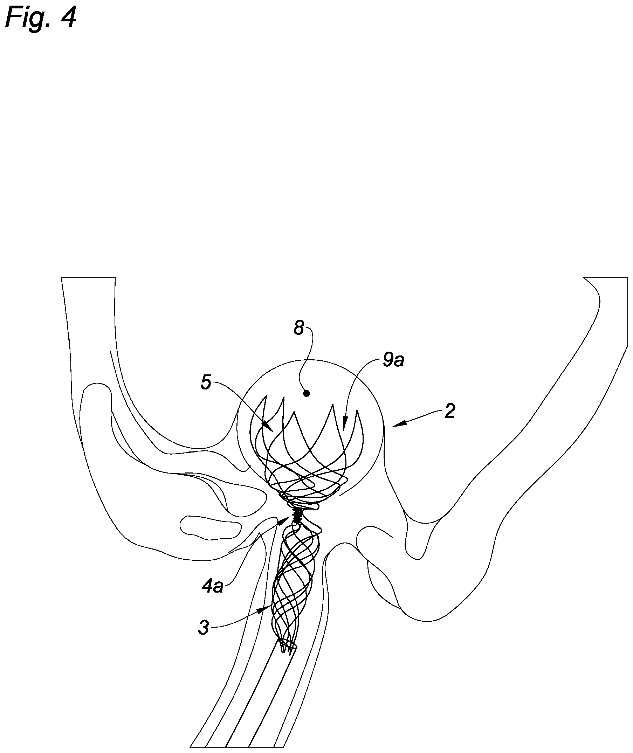

En référence à la figure 4, le dispositif 1 intra-anévrismal est illustré durant la phase finale de son déploiement dans un anévrisme 2 reconstitué pour les besoins des tests opérés. L'anévrisme 2 reconstitué est ici en silicone. Le dispositif 1 a conservé sa structure initiale. La tête 5 est correctement positionnée au sein de l'anévrisme 2. Elle repose sur le collet de l'anévrisme 2 et sa concavité 9a fait face à la poche anévrismale 8. La portion intermédiaire 4 est, elle, bien située au niveau dudit collet.

En référence à la figure 5, le résultat d'une simulation numérique de déploiement du dispositif 1 intra-anévrismal selon l'invention est illustré. Ces simulations, notamment la simulation illustrée à la figure 4b, permettent de mieux distinguer une extrémité de la portion proximale 3.

Dans ce qui suit sont décrits des procédés 60 de liaison des extrémités 6a des fils entre elles.

Précisons qu'avant de mettre en oeuvre ce procédé, on fournit le dispositif 1 intra-anévrismal comprenant la structure tressée S telle que décrite précédemment avec pour seule particularité que les extrémités 6a des fils ne sont pas jointes.

En référence à la figure 6, une vue rapprochée du dispositif 1 intra-anévrismal selon l'invention est illustrée aux fins de la compréhension des étapes du procédé de liaison desdites extrémités 6a desdits fils entre elles.

Lors d'une première étape 61, on rapproche deux fils 6 dont les extrémités 6a doivent être jointes de sorte que leurs extrémités 6a soient sensiblement parallèles.

En rapprochant les deux fils 6, on forme une portion de forme sensiblement triangulaire dont un sommet correspondant à un point de rapprochement (qui une fois définitivement soudé n'est autre que la portion 25 de liaison précédemment décrite) desdites extrémités 6a présente un angle α (visible sur les figures 6a et 6b).

De préférence, au sein du dispositif au repos, l'angle α est le plus fermé possible, c'est-à-dire que l'angle α n'est pas ouvert. La tête 5 peut ainsi être rétractée au minimum lors de son insertion dans le micro-cathéter 50 sans que ne se perdent concomitamment les liaisons entre les extrémités 6a desdits fils. En effet, en solidarisant les extrémités 6a avec un angle ouvert, les contraintes radiales exercées par les parois du micro-cathéter 50 sur la tête 5 provoqueraient une désolidarisation des extrémités 6a avant la fin de la phase de déploiement.

Lors d'une deuxième étape 62, on relie avantageusement certaines des extrémités 6a entre elles au moyen d'un procédé de fixation.

Avantageusement, cette liaison peut être effectuée par soudure des extrémités 6a sans ajout de matière. Dans ce cas, on fait fusionner les deux extrémités 6a par soudure en utilisant comme matériau pour la fusion le matériau aux extrémités 6a. La soudure sans ajout de matière permet de réduire l'encombrement du dispositif 1, ce qui facilite son insertion dans la zone touchée par l'anévrisme. Ici, le matériau dont il s'agit est de préférence un alliage de nickel-titane ou nitinol. Pour rappel, la structure tressée S peut avantageusement être faite en nitinol. Les avantages d'une telle matière sont rappelés dans les sections précédentes.

Si la portion 25 de liaison se présente sous forme d'une ligne ou d'un cordon, comme mentionné précédemment, on met en parallèle deux extrémités 6a de fils et on les fait fusionner par soudure en utilisant, comme dans la configuration précédemment décrite, le matériau des extrémités 6a. De préférence, cette fusion est opérée sur une longueur comprise entre 0,05 mm et 1 mm. Là encore, aucun ajout de matière n'est nécessaire, ce qui permet d'améliorer la résistance de la liaison mécanique entre deux extrémités 6a sans augmenter l'encombrement du dispositif.

De telles soudures pourraient par exemple être mises en oeuvre au moyen d'un résonateur SweetSpot ^{®}. Un tel dispositif permet avantageusement de réaliser des soudures de taille inférieure à 0,1 mm.

Cela étant, les liaisons aux extrémités peuvent également être réalisées par soudure avec ajout de matière. Dans ce cas, au lieu d'utiliser le matériau dont sont faites les extrémités 6a, la soudure est effectuée avec un apport de métal en fusion sur le point de rapprochement. À cet égard, on pourra avoir recours à un monobrin bleu de 1 à 2 mm.

La troisième étape 63 est une étape de refroidissement. Une fois que le point de soudure est complètement refroidi, les deux extrémités 6a sont définitivement liées. Le dispositif est alors prêt à être utilisé.

En remplacement des étapes 61 à 63 relatives à un procédé de soudure, on peut également prévoir un procédé de sertissage des extrémités 6a des fils.

## Revendications

1. Dispositif (1) intra-anévrismal pour le traitement d'un anévrisme (2), comprenant une structure tressée (S), à mailles, réalisée avec une pluralité de fils, la structure tressée (S) comprenant une tête (5) destinée à être insérée dans l'anévrisme (2), la tête (5) étant apte à réduire significativement un flux sanguin dans l'anévrisme (2), ladite tête (5) présentant une forme de coupole, la structure tressée (S) comprend au niveau d'une extrémité proximale de ladite structure tressée (S) un noeud (4a) relié à la tête (5) et, le dispositif (1) étant **caractérisé en ce que**, au niveau de son extrémité distale opposée à ladite extrémité proximale, la tête (5) est formée d'extrémités (6a) de fils dont certaines au moins sont liées mécaniquement entre elles, les extrémités des fils liés mécaniquement étant parallèles et liés deux à deux.

2. Dispositif (1) selon la revendication 1, dans lequel les extrémités (6a) de fils liées mécaniquement sont liées sur une ligne.

3. Dispositif (1) selon l'une des revendications 1 ou 2, dans lequel les mailles de la structure tressée (S) sont plus denses au niveau de l'extrémité proximale de ladite tête qu'au niveau de l'extrémité distale de ladite tête.

4. Dispositif (1) selon l'une quelconque des revendications précédentes, dans lequel la tête (5) présente une géométrie sensiblement demi-sphérique tournée vers l'extérieur, l'extrémité distale de ladite tête (5) présentant une section sensiblement circulaire de diamètre (D2), et le noeud (4a) présentant une section sensiblement circulaire dont un diamètre (D3) est inférieur au diamètre (D2) de la tête (5).

5. Dispositif (1) selon l'une des revendications précédentes, dans lequel le nombre de fils est compris entre 4 et 250, de préférence compris entre 16 et 32.

6. Dispositif (1) selon l'une quelconque des revendications précédentes, dans lequel les fils (6) présentent un diamètre compris entre 10 µm et 500 µm.

7. Dispositif (1) selon l'une quelconque des revendications précédentes, dans lequel les fils (6) se superposent au niveau de zones (27) d'intersection, lesdits fils (6) étant mécaniquement liés au niveau desdites zones (27) d'intersection.

8. Dispositif (1) selon l'une quelconque des revendications précédentes, dans lequel certaines au moins des extrémités (6a) de fils sont liées mécaniquement entre elles par l'intermédiaire d'une portion (25) de liaison faite d'une partie d'un premier fil (6) et d'une partie d'un deuxième fil (6).

9. Dispositif (1) selon l'une quelconque des revendications précédentes, dans lequel les fils (6) sont en matériau biocompatible de préférence du nitinol, platine ou titane.

10. Dispositif (1) selon l'une quelconque des revendications précédentes, dans lequel au moins un fil (6) est radio-opaque.

11. Dispositif (1) selon l'une quelconque des revendications précédentes comprenant une portion proximale (3) destinée à positionner le dispositif (1), ledit noeud (4a) reliant la portion proximale (3) à la tête (5).

12. Dispositif (1) selon l'une quelconque des revendications précédentes, dans lequel les mailles de la structure tressée au niveau du noeud (4a) sont plus denses que les mailles de la structure tressée au niveau de l'extrémité proximale de la tête.

13. Dispositif (1) selon l'une des revendications 1 à 11, dans lequel ledit noeud (4a) est formé par une bague de sertissage reliant des extrémités de fils (6c) entre elles.

14. Dispositif (1) selon l'une des revendications 1 à 11, dans lequel ledit noeud (4a) est formé par des extrémités de fils (6c) soudées entre elles.

## Patentansprüche

1. Intra-aneurysmatische Vorrichtung (1) zur Behandlung eines Aneurysmas (2), die eine geflochtene Struktur (S) mit Maschen umfasst, die mit einer Vielzahl von Drähten hergestellt ist, wobei die geflochtene Struktur (S) einen Kopf (5) umfasst, der bestimmt ist, in das Aneurysma (2) eingeführt zu werden, wobei der Kopf (5) dazu geeignet ist, einen Blutfluss im Aneurysma (2) erheblich zu reduzieren, wobei der Kopf (5) eine Kuppelform aufweist, wobei die geflochtene Struktur (S) im Bereich eines proximalen Endes der geflochtenen Struktur (S) einen Knoten (4a) umfasst, der mit dem Kopf (5) verbunden ist, und wobei die Vorrichtung (1) **dadurch gekennzeichnet ist, dass** der Kopf (5) im Bereich seines distalen Endes gegenüber dem proximalen Ende aus Drahtenden (6a) besteht, von denen zumindest einige mechanisch miteinander verbunden sind, wobei die mechanisch miteinander verbundenen Drahtenden parallel und paarweise verbunden sind.

2. Vorrichtung (1) nach Anspruch 1, wobei die mechanisch verbundenen Drahtenden (6a) in einer Reihe verbunden sind.

3. Vorrichtung (1) nach einem der Ansprüche 1 oder 2, wobei die Maschen der geflochtenen Struktur (S) im Bereich des proximalen Endes des Kopfes dichter sind als im Bereich des distalen Endes des Kopfes.

4. Vorrichtung (1) nach einem der vorhergehenden Ansprüche, wobei der Kopf (5) eine im Wesentlichen halbkugelförmige, nach außen gerichtete Geometrie aufweist, wobei das distale Ende des Kopfes (5) einen im Wesentlichen kreisförmigen Querschnitt mit Durchmesser (D2) aufweist und der Knoten (4a) einen im Wesentlichen kreisförmigen Querschnitt aufweist, dessen Durchmesser (D3) kleiner ist als der Durchmesser (D2) des Kopfes (5).

5. Vorrichtung (1) nach einem der vorhergehenden Ansprüche, wobei die Anzahl der Drähte zwischen 4 und 250, vorzugsweise zwischen 16 und 32 liegt.

6. Vorrichtung (1) nach einem der vorhergehenden Ansprüche, wobei die Drähte (6) einen Durchmesser zwischen 10 µm und 500 µm aufweisen.

7. Vorrichtung (1) nach einem der vorhergehenden Ansprüche, wobei sich die Drähte (6) im Bereich der Schnittpunktzonen (27) überlappen, wobei die Drähte (6) im Bereich der Schnittpunktzonen (27) mechanisch verbunden sind.

8. Vorrichtung (1) nach einem der vorhergehenden Ansprüche, wobei einige zumindest der Drahtenden (6a) durch einen Verbindungsabschnitt (25), der aus einem Teil eines ersten Drahtes (6) und einem Teil eines zweiten Drahtes (6) besteht, mechanisch miteinander verbunden sind.

9. Vorrichtung (1) nach einem der vorhergehenden Ansprüche, wobei die Drähte (6) aus einem biokompatiblen Material bestehen, vorzugsweise aus Nitinol, Platin oder Titan.

10. Vorrichtung (1) nach einem der vorhergehenden Ansprüche, wobei zumindest ein Draht (6) röntgendicht ist.

11. Vorrichtung (1) nach einem der vorhergehenden Ansprüche, die einen proximalen Abschnitt (3) zur Positionierung der Vorrichtung (1) umfasst, wobei der Knoten (4a) den proximalen Abschnitt (3) mit dem Kopf (5) verbindet.

12. Vorrichtung (1) nach einem der vorhergehenden Ansprüche, wobei die Maschen der geflochtenen Struktur im Bereich des Knotens (4a) dichter sind als die Maschen der geflochtenen Struktur im Bereich des proximalen Endes des Kopfes.

13. Vorrichtung (1) nach einem der Ansprüche 1 bis 11, wobei der Knoten (4a) durch einen Quetschring gebildet ist, der Drahtenden (6c) miteinander verbindet.

14. Vorrichtung (1) nach einem der Ansprüche 1 bis 11, wobei der Knoten (4a) durch miteinander verschweißte Drahtenden (6c) gebildet ist.

## Claims

1. Intra-aneurysm device (1) for the treatment of an aneurysm (2), comprising a braided structure (S), with mesh, made with a plurality of wires, the braided structure (S) comprising a head (5) intended to be inserted into the aneurysm (2), the head (5) being able to significantly reduce blood flow in the aneurysm (2), said head (5) having a dome shape, the device (1) being **characterised in that** the braided structure (S) comprises at a proximal end of said braided structure (S) a node (4a) connected to the head (5), and **in that**, at its distal end opposite to said proximal end, the head (5) is formed of ends (6a) of wires, at least some of which are mechanically connected together.

2. Device (1) according to claim 1, wherein the wire ends (6a) are mechanically connected at least in pairs.

3. Device (1) according to one of claims 1 or 2, wherein the meshes of the braided structure (S) are denser at the proximal end of said head than at the distal end of said head.

4. Device (1) according to any one of the preceding claims, wherein the head (5) has a substantially semi-spherical geometry facing outwards, the distal end of said head (5) having a substantially circular section of diameter (D2), and the node (4a) having a substantially circular section whose diameter (D3) is smaller than the diameter (D2) of the head (5).

5. Device (1) according to one of the preceding claims, wherein the number of wires is between 4 and 250, preferably between 16 and 32.

6. Device (1) according to any one of the preceding claims, wherein the wires (6) have a diameter of between 10 µm and 500 µm.

7. Device (1) according to any one of the preceding claims, wherein the wires (6) are superimposed at the location of areas (27) of intersection, said wires (6) being mechanically connected at the location of said areas (27) of intersection.

8. Device (1) according to any one of the preceding claims, wherein at least some of the wire ends (6a) are mechanically connected together by means of a connecting portion (25) made of a portion of a first wire (6) and a portion of a second wire (6).

9. Device (1) according to any one of the preceding claims, wherein the wires (6) are made of a biocompatible material, preferably nitinol, platinum or titanium.

10. Device (1) according to any one of the preceding claims, wherein at least one wire (6) is radiopaque.

11. Device (1) according to any one of the preceding claims comprising a proximal portion (3) intended to position the device (1), said node (4a) connecting the proximal portion (3) to the head (5).

12. Device (1) according to any one of the preceding claims, wherein the meshes of the braided structure at the node (4a) are denser than the meshes of the braided structure at the proximal end of the head.

13. Device (1) according to one of claims 1 to 11, wherein said node (4a) is formed by a crimping ring connecting wire ends (6c) together.

14. Device (1) according to one of claims 1 to 11, wherein said node (4a) is formed by wire ends (6c) welded together.
